# EUROPEAN PATENT SPECIFICATION

(11) **EP 3 608 870 B1**
(45) Date of publication and mention of the grant of the patent: **24.12.2025**
(21) Application number: 18188557.5
(22) Date of filing: 10.08.2018
(51) Int. Cl.: G06T 7/10, A61B 34/10, A61B 90/00

(54) **COMPUTER ASSISTED IDENTIFICATION OF APPROPRIATE ANATOMICAL STRUCTURE FOR MEDICAL DEVICE PLACEMENT**
COMPUTERGESTÜTZTE IDENTIFIZIERUNG EINER GEEIGNETEN ANATOMISCHEN STRUKTUR FÜR DIE PLATZIERUNG VON MEDIZINISCHEN VORRICHTUNGEN
IDENTIFICATION ASSISTÉE PAR ORDINATEUR D'UNE STRUCTURE ANATOMIQUE APPROPRIÉE POUR LE PLACEMENT D'UN DISPOSITIF MÉDICAL

(43) Date of publication of application: 12.02.2020
(73) Proprietor: Augmedics Inc., Arlington Heights, IL 60005 (US)
(72) Inventor: Siemionow, Krzysztof B., Chicago, Illinois 60610 (US); Luciano, Cristian J., Evergreen Park, 60805 (US); Gawel, Dominik, 01-949 Warszawa (PL); Kraft, Marek, 61-251 Poznan (PL); Trzmiel, Michal, 01-949 Warszawa (PL); Fularz, Michal, 01-949 Warszawa (PL); Edwing, Isaac Mejia Orozco, 01-949 Warszawa (PL); Pietruszka, Michal, 01-949 Warszawa (PL)
(74) Representative: Kancelaria Eupatent.pl Sp. z o.o.

(56) References cited:
- WO-A1-2018/048575
- US-A1- 2013 226 190
- US-A1- 2019 029 757

## Description

### TECHNICAL FIELD

The present disclosure relates to computer assisted surgical navigation systems, in particular to a system and method for identifying appropriate anatomical structure for placement of a medical device, the medical device being an implant, during a surgical procedure, in particular related to neurological and general surgery procedures.

### BACKGROUND

Image guided or computer assisted surgery is a surgical procedure where the surgeon uses trackable surgical instruments, combined with preoperative or intraoperative images (e.g., from computer tomography (CT) scanners), in order to provide the surgeon with surgical guidance during the procedure. A PCT application WO2006096702 discloses a method of determining the size and/or placement of bone anchors or other instruments in pedicles during surgery in a selected spinal area, comprising generating a three-dimensional image of the bony spine in the selected spinal area; hollowing out the vertebra in the three-dimensional image with cortical wall thicknesses selected by a surgeon performing the surgery; determining the narrowest cross section (isthmus) within each pedicle; generating a straight line starting at the center of the isthmus and extending inwardly to a point centered within the anterior cortex so that it is positioned concentrically within the pedicle without touching the walls thereof, the line terminating inside the vertebral body a predetermined distance from the anterior inner cortical wall and extending outwardly in the opposite direction to penetrate the posterior pedicle cortex; expanding the line concentrically and radially to a cross sectional size that is less than that of the isthmus, the line being expanded into a cylinder that stops growing when any portion thereof contacts the inner cortical wall of the hollowed out vertebral body, with the exception of the posterior pedicle cortex; and calculating the ideal pedicle screw or instrument diameter, length and/or trajectory based on the dimensions and trajectory of the cylinder generated for each pedicle. Also, a new and improved method for providing access to the interior of a pedicle for a desired transpedicular procedure, and a new and improved pedicle cannula construction are disclosed herein.

A US patent application US20170112575 discloses a framework for pedicle screw positioning, wherein the framework segments at least one vertebra of interest in image data. The framework then automatically determines a pedicle region within the segmented vertebra of interest, and a safe region within the segmented vertebra of interest. An optimal insertion path passing through the pedicle region may then be generated within the safe region.

A PCT patent application WO2008038284 discloses a system and method for generating three dimensional CT-type information from a conventional C-arm fluoroscopy imaging system. A small number of fluoroscopy images are used, taken from angles whose pose is determined by means of a three-dimensional target attached to the region of interest, aided by the participation of the surgeon or an image processing routine to pinpoint known anatomical features in the region of interest of the patient. This procedure enables the reconstruction of virtual images in any desired plane, even in planes other than those accessible by the C-arm imaging process, such as the axial plane of a vertebra. Use of this system and method of marking of the feature to be treated in a small number of angularly dissimilar images, enables the generation of CT-type information which can be used to accurately align a robotically guided surgical tool with the anatomical feature.

A European patent application EP3120797 discloses identifying, with the aid of a computer and on an x-ray, or on a three-dimensional reconstruction by means of CT or Nuclear Magnetic Resonance (NMR), generated by a computer, the optimal direction and the maximum diameter of a pedicle screw so that the screw will not come out of the pedicle.

A US patent application US2008147173 discloses that one or more localization markers are implanted in a patient's vessel to provide a virtual image of a portion of the vessel, provide a target for deploying a prosthesis, and/or facilitate post-operative surveillance of a deployed prosthesis. In the case of providing a target or facilitating surveillance, one or more localization markers also can be provided on the prosthesis (e.g., the proximal end portion of the prosthesis) and the position of the prosthesis marker(s) monitored relative to the implanted marker(s).

A US patent US9592138 discloses devices, systems and methods for improving airflow within an airway. One example embodiment includes a method for treating a subject. The method includes (1) placing an expandable object into one or more airways of the bronchial tree of the subject, (2) expanding the expandable object within at least one of the one or more airways such that at least a portion of a wall of the one or more airways is expanded, and (3) placing a stent in the airway such that a portion of the stent is adjacent to the portion of the wall of the one or more expanded airways.

A US patent application US2017095314 discloses a kit for performing a reduced radiation percutaneous procedure. The kit includes a needle access device having a needle connected to a hub portion having an opaque cap portion, a non-opaque body portion positioned between the opaque cap portion and the needle, and a channel extending through the opaque cap portion; a sticker having an adhesive side adapted to adhere to the skin of a patient, and a display surface opposite the adhesive side configured to enhance visualization of the sticker in low light; and a guidewire having a floppy portion with a distal end, an intermediate region connected to the floppy portion, such that the intermediate region is less floppy than the floppy portion; and an ultrasonic-profile-enhancing feature disposed within 3 centimeters of the distal end of the floppy portion.

A Korean patent KR101687919 discloses a catheter surgery navigation system for balloon sinuplasty that includes: a catheter including the expansion and washing tubes and including far and near sections; an expandable balloon positioned in the far section of the catheter and connected to the expansion tube; a washing tip positioned in a far end portion of the catheter and including a tip opening portion connected to the washing tube; an electro-magnetic (EM) marker positioned between the balloon and the tip opening portion and generating a magnetic field signal; a position tracking unit grasping the positional information of the EM marker by using the magnetic field signal; and a control unit matching a pre-stored sinus image with the position of the EM marker by using the positional information.

A US patent application US20170312499 discloses devices, systems and methods for accessing and treating anatomies associated with a variety of conditions while minimizing possible complications and side effects by directly neuromodulating a target anatomy associated with the condition while minimizing or excluding undesired neuromodulation of other anatomies. In particular, some embodiments are used to selectively stimulate portions of the spinal nerves, particularly one or more dorsal root ganglions (DRGs), to treat chronic pain while causing minimal deleterious side effects such as undesired motor responses.

A PCT application WO2018048575 discloses systems and methods for developing radiotherapy treatment plans though the use of machine learning approaches and neural network components. A neural network is trained using one or more three-dimensional medical images, one or more three-dimensional anatomy maps, and one or more dose distributions to predict a fluence map or a dose map.

A US patent application US2013226190 discloses systems and methods for optimizing parameters of an orthopaedic procedure for a particular patient, including parameters relating to the anatomic and biomechanic fit of an implant or implant system implanted into the patient's joint. These systems and methods may utilize patient-specific information gathered preoperatively in conjunction with optimization algorithms to determine an optimal implant design and an optimal position and orientation for implantation of the implant into the particular patient's joint.

The disadvantage of the solutions discussed above is that they are not fully automatic. According to the patent descriptions, the methods require a specialized person to analyze the X-Ray, CT or NMR data and select a starting point for the procedure. Moreover the patents do not mention anything about the intraoperative CT allowing proper positioning during the surgery. In comparison, the invention presented herein allows for fully automatic positioning and size determination in the 3D domain of the ongoing surgery thanks to usage of an intraoperative scanner and Artificial-Intelligence-based methods.

### SUMMARY

There is disclosed herein a method and a system for computer assisted identification of appropriate anatomical structure for placement of a medical device according to the appended claims.

### BRIEF DESCRIPTION OF DRAWINGS

The present invention is shown by means of example embodiments on a drawing, wherein:
Fig. 1 shows an overview of a training procedure;
Figs. 2A-2C show images used in the system during the procedures;
Fig. 2D shows region of interest used in the process;
Fig. 2E shows 3 dimensional resizing of region of interest;
Fig. 2F shows exemplary characteristic features localization;
Fig. 2G shows exemplary results of artificial training database augmentation;
Fig. 2H shows exemplary final implant localization;
Fig. 3 shows an overview of a prediction procedure;
Fig. 4 shows a prediction CNN architecture;
Fig. 5 shows a flowchart of a training process for the prediction CNN;
Fig. 6 shows a flowchart of an inference process for the prediction CNN;

### DETAILED DESCRIPTION

The term "medical device" is understood as a surgical implant or an instrument, for example a catheter, instrument, a cannula, a needle, a anchor, a screw, a stent, a biomechanical device.

The present disclosure will be described in details with reference to an example embodiment related to a neurological surgery, wherein a screw (as an example of a medical device) is placed, i.e. inserted, to a spine (as an example of an anatomical structure). A skilled person will realize that this embodiment can be extended to other applications as well, such as guidance for a medical device (e.g., instrumentation or implant) in other natural or artificial anatomical structures, for example blood vessels, biliary ducts, subthalamic nucleus, and components of solid organs like the heart (e.g., mitral valve), kidney (e.g., renal artery), and nerves (e.g., epidural space).

The automatic implant placement method as presented herein comprises two main procedures: a training procedure and a prediction procedure.

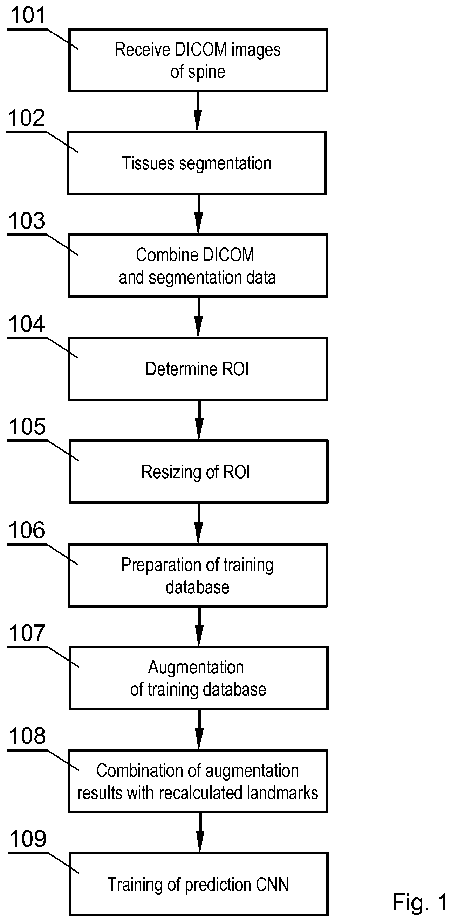

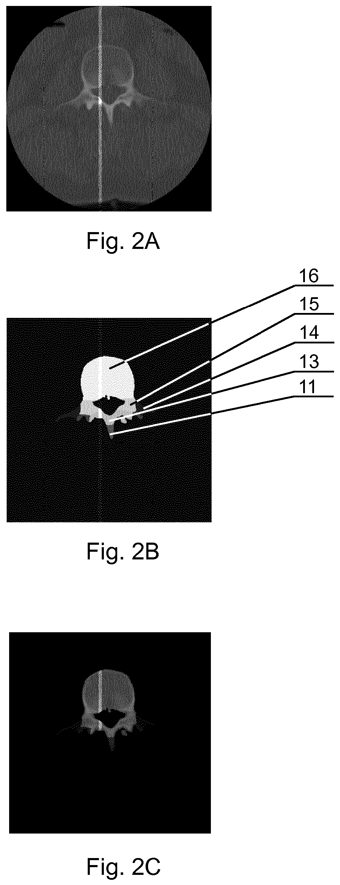

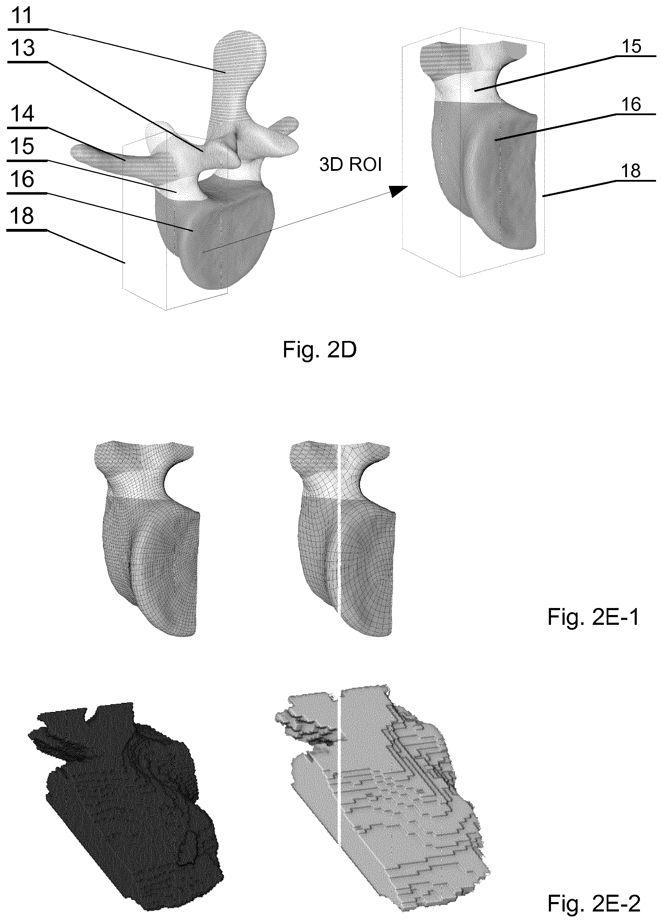

The training procedure, as presented in Fig. 1, comprises the following steps. First, in step 101, a set of DICOM (Digital Imaging and Communications in Medicine) images obtained with a preoperative or an intraoperative CT (Computed Tomography) or MRI (Magnetic Resonance Imaging) representing consecutive slices with visible tissues is received (such as one slice shown in Fig. 2A). Next, the received images are processed in step 102 to perform automatic segmentation of tissues, such as to determine separate areas corresponding to different tissues, such as vertebral body 16, pedicles 15, transverse processes 14 and/or spinous process 11, as shown in Fig. 2B. For example, this can be done by employing a method for segmentation of images disclosed in a European patent application EP17195826 by the present applicant. Then, in step 103, the information obtained from DICOM images and the segmentation results is merged to obtain combined image comprising information about the tissue appearance and its classification (including assignment of structure parts to classes corresponding to different anatomy parts), for example in a form of a color-coded DICOM image, as shown in Fig. 2C. Alternatively, separate DICOM (Fig. 2A) and segmentation (Fig. 2B) images can be processed instead of the combined image. Next, in step 104, from the set of slice images a 3D region of interest (ROI) 18 is determined, that contains a volume of each pedicle 15 with a part of adjacent vertebral body 16 and surrounding tissues such as lamina 13, transverse process 14 and others, as shown in Fig. 2D. Then, in step 105, the 3D resizing of the determined ROI 18 is performed to achieve the same size of all ROI's stacked in the 3D matrices, each containing information about voxel distribution along X, Y and Z axes and the appearance and classification information data for each voxel, such as shown in Fig. 2E-1 or 2E-2. In other words, the voxels are small cuboidal volumes resembling points having 3D coordinates and a radio density value and classification assigned.

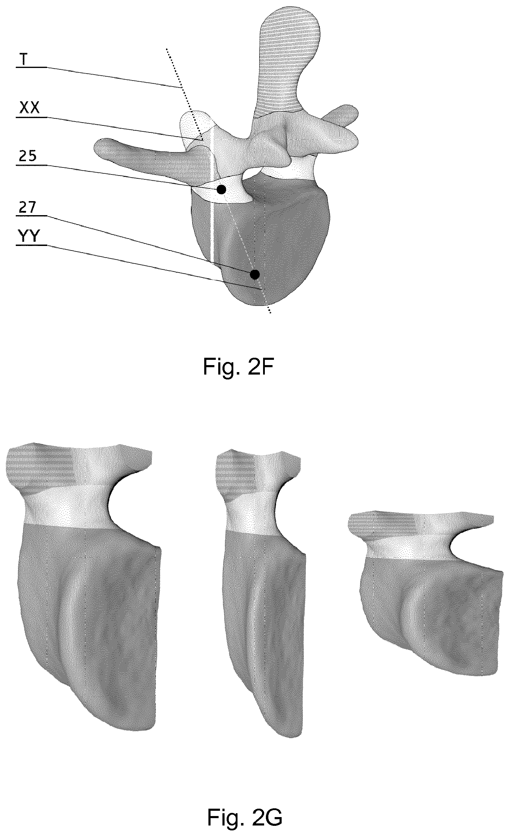

Next, in step 106, a training database is prepared manually, that comprises the previously determined ROIs and manually landmarked characteristic features corresponding to pedicle center 25 and screw tip 27 (or other anatomical structure and device points), such as shown in Fig. 2F.

Next, in step 107, the training database is augmented, for example with the use of a 3D generic geometrical transformation and resizing with random dense 3D grid deformations, as shown in Fig. 2G. Data augmentation is performed on the images to make the training set more diverse. The foregoing transformations are remapping the voxels positions in a 3D ROI 18 based on a randomly warped artificial grid assigned to the ROI 18 volume. A new set of voxel positions is calculated artificially warping the 3D tissue shape and appearance. Simultaneously, the information about the tissue classification is warped to match the new tissue shape and the manually determined landmarks positions 25, 27 are recalculated in the same manner. During the process, the value of each voxel, containing information about the tissue appearance, is recalculated in regards to its new position in ROI 18 with use of an interpolation algorithm (for example bicubic, polynomial, spline, nearest neighbor, or any other interpolation algorithm) over the 3D voxel neighborhood.

Next, in step 108, the obtained artificial database augmentation results are combined with the automatically recalculated landmarks, corresponding to the artificially augmented pedicle centers 25 and screw tips 27 (or other anatomical structure and device points), into a single database interpretable by a neural network.

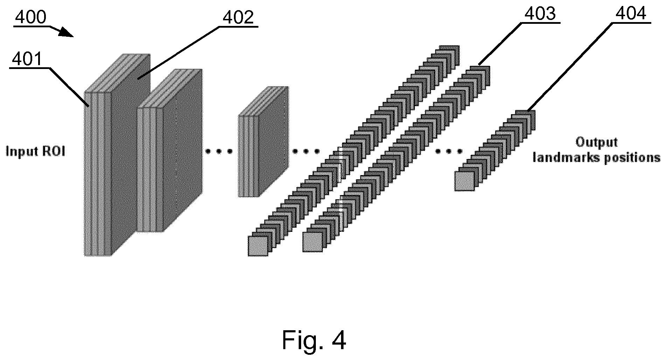

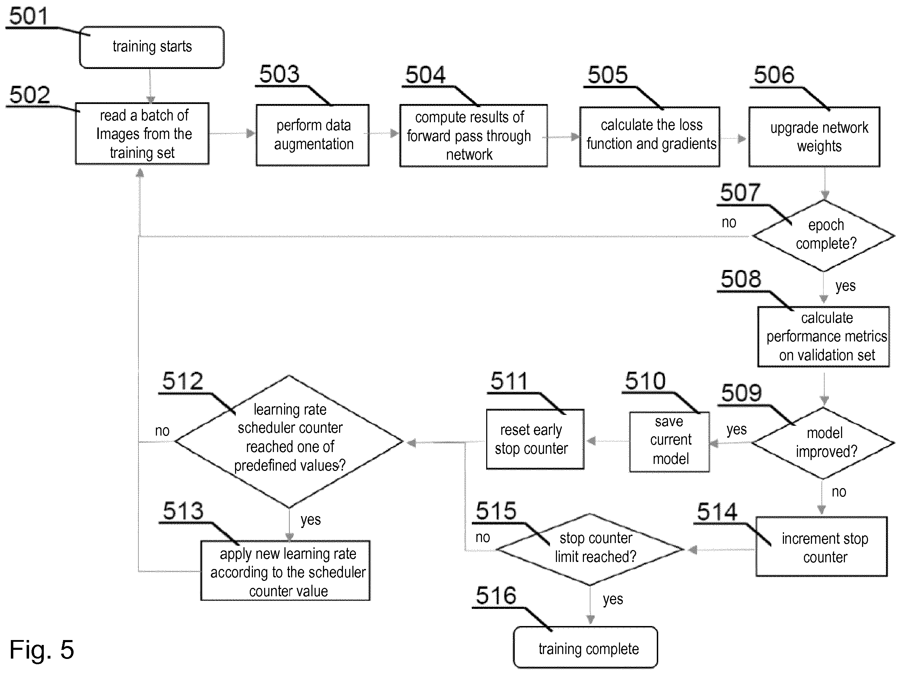

Then, in step 109, the placement prediction model is trained with a neural network. Preferably, a network with a plurality of layers can be used, specifically a combination of convolutional and fully connected layers with ReLU activation functions or any other non-linear or linear activation functions. For example, a network such as shown in Fig. 4, according to a process such as shown in Fig. 5, can be used.

The training database may also comprise data from actually performed surgical procedures. The system may automatically identify and store the 3D position and orientation of the medical device actually inserted by the surgeon in the anatomical structure during the surgical procedure, for further training the prediction neural network (400) in order to improve its performance to subsequently identify the preferred positions and orientations. Therefore, the system may operate like a closed feedback loop.

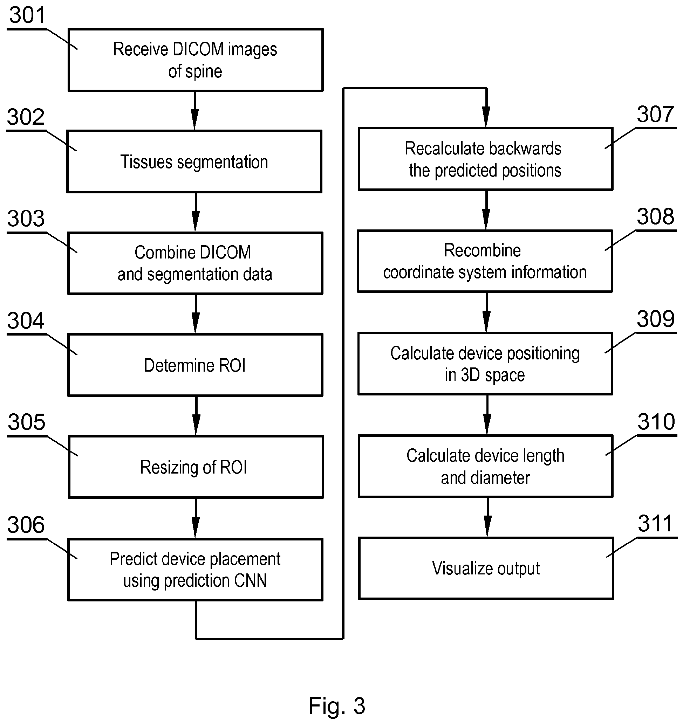

The prediction procedure, as presented in Fig. 3, comprises the following steps. First, in step 301, a 3D scan volume is received, comprising a set of DICOM (Digital Imaging and Communications in Medicine) images of a region of the anatomical structure where the medical device is to be placed. The 3D scan volume can be obtained with a preoperative or an intraoperative CT (Computed Tomography) or MRI (Magnetic Resonance Imaging). The set of DICOMs representing consecutive slices of a spine is received (such as one slice shown in Fig. 2A). Next, the received images are processed in step 302 to perform automatic segmentation of tissues of the anatomical structure, such as to determine separate areas corresponding to different tissues, such as vertebral body 16, pedicles 15, transverse processes 14, lamina 13 and/or spinous process 11, as shown in Fig. 2B. For example, this can be done by employing a method for segmentation of images disclosed in a European patent application EP17195826 by the present applicant. Then, in step 303, the information obtained from DICOM images and the segmentation results is merged to obtain combined image comprising information about the tissue appearance and its classification, for example in a form of a color-coded DICOM image, as shown in Fig. 2C. Alternatively, separate DICOM (Fig. 2A) and segmentation (Fig. 2B) images can be processed instead of the combined image. Next, in step 304, from the 3D scan volume a 3D region of interest (ROI) 18 is automatically determined. For example, the ROI 18 may contain a volume of each pedicle 15 with a part of adjacent vertebral body and surrounding tissues, as shown in Fig. 2D. Then, in step 305, the 3D resizing of the determined ROI 18 is performed to achieve the same size of all ROI's stacked in the 3D matrices. Each 3D matrix contains information about voxel distribution along X, Y and Z axes with bone density and classification information data for each voxel, such as shown in Fig. 2E. Therefore, steps 301-305 are performed in a way similar to steps 101-105 of the training procedure of Fig. 1.

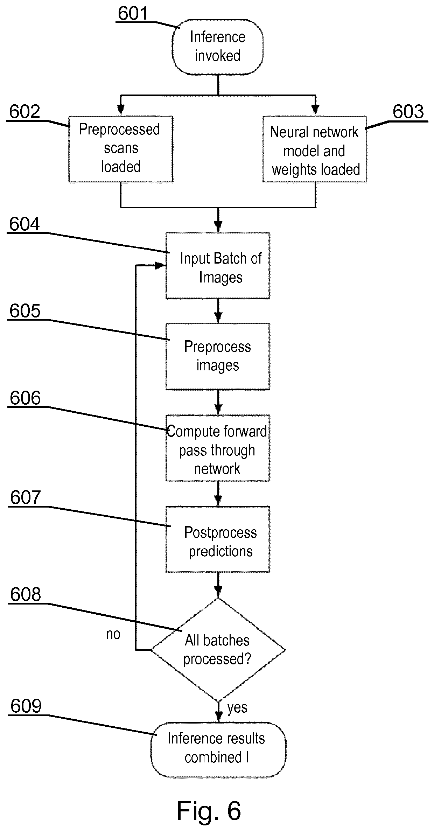

Next, in step 306, the preferred placement is predicted automatically by processing the resized ROI to determine the preferred 3D position and orientation of the medical device to be placed with respect to the anatomical structure, by means of the pretrained prediction CNN 400, according to the prediction process presented in Fig. 6. The prediction CNN 400 is configured to identify landmarks within the anatomical structure, such as pedicle center 25 and screw tip 27.

Next, in step 307, the predicted screw tip 25 and pedicle center 27(or other anatomical structure and device landmarks) positions within the ROI are backward recalculated to meet the original ROI size and positions from input DICOM dataset to recreate and ensure a correct placement in original volume.

In step 308 the information about the global coordinate system (ROI position in the DICOM dataset) and local ROI coordinate system (predicted screw tip and pedicle center positions inside the ROI) is recombined.

Then, in step 309, the preferred device positioning in the 3D space is calculated, based on two landmarks corresponding to pedicle center 25 and screw tip 27, as shown in Fig. 2F.

Anatomical knowledge and preferred device positioning allow for the calculation of a preferred device's physical dimensions, for example screw positioning in the vertebra. With the semantic/anatomical segmentation results and pedicle center 25 location available, in step 310, automated computation of device physical dimensions, such as the diameter, is possible. Proceeding in the coronal direction, forward and backward from the pedicle center landmark 25 along the pedicle, the slice for which the inscribed circle diameter will be the smallest can easily be found. A fraction of this diameter corresponds directly to the inserted device maximum allowed diameter with a necessary safety margin, that can be easily defined by the user of the system.

Enabling selection of a specific element in the available series of types also requires determination of device physical dimensions such as the length. This too can be easily computed automatically using the device insertion trajectory information provided by the neural network. The line going through the estimated landmarks (bone anchor tip 27, pedicle center 25) represents the trajectory of the device, which can be expressed as a 3D path, in the case of 2 landmarks it will be line model. Given the trajectory of a medical device to be inserted and an anatomical structure being a target, the entry and exit points could be calculated using automated 3D image analysis. For example, given the 3D line model and a 3D shell of the shape of the anatomical part being a target of device insertion extracted using morphological gradient in 3D (a single voxel thick surface of all solids in the volume), the entry and exit points of the trajectory are located at the two shell voxels (XX, YY) that are closest to the line (trajectory T) at each end, for example such as shown in Fig. 2F.

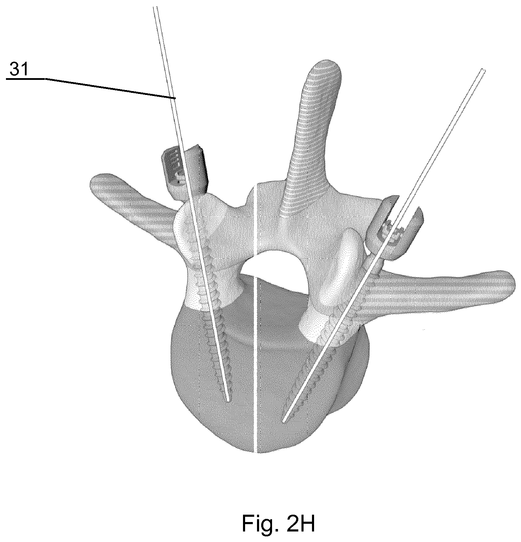

Next, in step 311, the output is visualized, for example such as shown in Fig. 2H, including the device 31 to be inserted.

Fig. 4 shows a convolutional neural network (CNN) architecture 400, hereinafter called the prediction CNN, which is utilized in the present method for prediction of device placement. The network performs device localization task using at least one input as a 3D information about the appearance (radiodensity) and the classification for each voxel in a 3D ROI.

The left side of the network is a contracting path, which includes convolution layers 401 and pooling layers 402, and the right side is a regression path which includes fully connected layers 403 and the output layer 404.

One or more 3D ROI's can be presented to the input layer of the network to learn reasoning from the data.

The convolution layers 401 can be of a standard kind, the dilated kind, or a combination thereof, with ReLU, leaky ReLU or any other kind of activation function attached.

The fully connected layers 403 can have Linear, ReLU or any other kind of activation function attached.

The output layer 404 also denotes the fully connected layer with the loss function, for example the loss function can be implemented as mean squared error or another metric.

The architecture is general, in the sense that adopting it to ROI's of different size is possible by adjusting the size (resolution) of the layers. The number of layers and number of filters within a layer is also subject to change, depending on the requirements of the application, for example as presented in the European patent application EP17195826.

The final layer for the device placement defines the preferred device position and orientation along X, Y and Z axes in 3D ROI. Prediction is based on the model trained from the manually prepared examples during the training process, for example in case of screw insertion, preferred position of the pedicle center 25 and screw tip 27.

Fig. 5 shows a flowchart of a training process, which can be used to train the prediction CNN 400. The objective of the training for the prediction CNN 400 is to tune the parameters of the prediction CNN 400 such that the network is able to predict preferred guidance for the device.

The training database may be separated into a training set used to train the model, a validation set used to quantify the quality of the model, and a test set.

The training starts at 501. At 502, batches of training ROI's are read from the training set, one batch at a time.

At 503 the ROI's can be additionally augmented. Data augmentation is performed on these ROI's to make the training set more diverse. The input/output data is subjected to the combination of transformations from the following set: rotation, scaling, movement, horizontal flip, additive noise of Gaussian and/or Poisson distribution and Gaussian blur, volumetric grid deformation, etc. or could be augmented with the use of generative algorithm such as Generative Adversarial Networks for example.

At 504, the ROI's are then passed through the layers of the CNN in a standard forward pass. The forward pass returns the results, which are then used to calculate at 505 the value of the loss function - the difference between the desired and the computed outputs. The difference can be expressed using a similarity metric (e.g., mean squared error, mean average error or another metric).

At 506, weights are updated as per the specified optimizer and optimizer learning rate using Gradient Descent methods (e.g., Stochastic Gradient Descent, Adam, Nadam, Adagrad, Adadelta, RMSprop).

The loss is also back-propagated through the network, and the gradients are computed. Based on the gradient values, the network's weights are updated. The process (beginning with the ROI's batch read) is repeated continuously until the end of the training session is reached at 507.

Then, at 508, the performance metrics are calculated using a validation dataset - which is not explicitly used in training set. This is done in order to check at 509 whether or not the model has improved. If it is not the case, the early stop counter is incremented at 514 and it is checked at 515 if its value has reached a predefined number of epochs. If so, then the training process is complete at 516, since the model has not improved for many sessions now.

If the model has improved, the model is saved at 510 for further use and the early stop counter is reset at 511. As the final step in a session, learning rate scheduling can be applied. The session at which the rate is to be changed are predefined. Once one of the session numbers is reached at 512, the learning rate is set to one associated with this specific session number at 513.

Once the training is complete, the network can be used for inference (i.e., utilizing a trained model for prediction on new data).

Fig. 6 shows a flowchart of an inference process for the prediction CNN 400.

After inference is invoked at 601, a set of ROI's is loaded at 602 and the prediction CNN

400 and its weights are loaded at 603.

At 604, one batch of ROI's at a time is processed by the inference server.

At 605, the images can be preprocessed (e.g., normalized)

At 606, a forward pass through the prediction CNN 400 is computed.

At 607, a postprocess prediction is done.

At 608, if not all batches have been processed, a new batch is added to the processing pipeline until inference has been performed on all input ROI's.

Finally, at 609, the inference results are saved and can be recalculated to provide an output in a form of preferred device position.

The functionality described herein can be implemented in a computer system. The system may include at least one nontransitory processor-readable storage medium that stores at least one of processor-executable instructions or data and at least one processor communicably coupled to that at least one nontransitory processor-readable storage medium. That at least one processor is configured to perform the steps of the method presented herein.

## Claims

1. A method for computer assisted identification of appropriate anatomical structure for implant placement of a medical device in the anatomical structure, wherein the medical device is a surgical implant, comprising the steps of:
- receiving a 3D scan volume comprising set of medical scan images of a region of an anatomical structure where the medical device is to be implanted (301);
- automatically processing the set of medical scan images to obtain segmentation data (302);
- combining the set of medical scan images with the segmentation data into combined images (303);
- automatically determining a a subsection of the 3D scan volume as a 3D region of interest (ROI) (304) from the combined images ;
- automatically processing the 3D ROI to identify landmarks within the anatomical structure for implanting the medical device in the anatomical structure using a pre-trained prediction convolutional neural network CNN (400);
- automatically determining, based on the landmarks, a preferred 3D position and orientation of the medical device to be implanted with respect to the anatomical structure (309); and
- automatically determining a preferred 3D position and orientation of the medical device to be implanted with respect to the 3D scan volume of the anatomical structure (309).

2. The method according to claim 1, further comprising:
- receiving, from a training database, data from an actually performed surgical procedure involving implanting a medical device by a surgeon in the anatomical structure during the surgical procedure,
- automatically identifying and storing a 3D position and orientation of the medical device actually implanted by the surgeon in the anatomical structure,
- and using the 3D position and orientation of the medical device actually implanted by the surgeon for further training of the prediction neural network (400) to improve an accuracy of the prediction neural network (400) to subsequently identify a preferred position and orientation.

3. The method according to claim 1, further compromising processing the scan images of the anatomical structures between the identified landmarks, and determining physical dimensions of the anatomical structures in the region of interest where the medical device is intended to be implanted.

4. The method according to claim 3, further comprising determining preferred physical dimensions, the preferred physical dimensions including at least one of: diameter and/or length, of the medical device to be implanted depending on analyzed dimensions of the anatomical structure.

5. The method according to claim 1, wherein the received medical scan images are collected from an intraoperative scanner.

6. The method according to claim 1, wherein the received medical scan images are collected from a presurgical stationary scanner.

7. A computer-implemented system, comprising:
- at least one nontransitory processor-readable storage medium that stores at least one of processor-executable instructions or data; and
- at least one processor communicably coupled to at least one nontransitory processor-readable storage medium, wherein at least one processor is configured to perform the steps of the method of any of previous claims

## Patentansprüche

1. Verfahren zur computergestützten Identifizierung einer geeigneten anatomischen Struktur zur Implantatplatzierung einer medizinischen Vorrichtung in der anatomischen Struktur, wobei die medizinische Vorrichtung ein chirurgisches Implantat ist, umfassend die folgenden Schritte:
- Empfangen eines 3D-Abtastvolumens, das einen Satz von medizinischen Abtastbildern einer Region einer anatomischen Struktur umfasst, in die die medizinische Vorrichtung zu implantieren ist (301);
- automatisches Verarbeiten des Satzes von medizinischen Abtastbildern, um Segmentierungsdaten zu erhalten (302);
- Kombinieren des Satzes von medizinischen Abtastbildern mit den Segmentierungsdaten zu kombinierten Bildern (303);
- automatisches Bestimmen eines Teilabschnitts des 3D-Abtastvolumens als eine 3D-Region von Interesse (ROI) (304) aus den kombinierten Bildern;
- automatisches Verarbeiten der 3D-ROI, um Orientierungspunkte innerhalb der anatomischen Struktur zum Implantieren der medizinischen Vorrichtung in die anatomische Struktur unter Verwendung eines vorab trainierten neuronalen Vorhersagefaltungsnetzwerks CNN (400) zu identifizieren;
- automatisches Bestimmen, basierend auf den Orientierungspunkten, einer bevorzugten 3D-Position und -Ausrichtung der zu implantierenden medizinischen Vorrichtung in Bezug auf die anatomische Struktur (309); und
- automatisches Bestimmen einer bevorzugten 3D-Position und -Ausrichtung der zu implantierenden medizinischen Vorrichtung in Bezug auf das 3D-Abtastvolumen der anatomischen Struktur (309).

2. Verfahren nach Anspruch 1, ferner umfassend:
- Empfangen, aus einer Trainingsdatenbank, von Daten von einem tatsächlich durchgeführten chirurgischen Eingriff, der Implantieren einer medizinischen Vorrichtung durch einen Chirurgen in die anatomische Struktur während des chirurgischen Eingriffs einbezieht,
- automatisches Identifizieren und Speichern einer 3D-Position und -Ausrichtung der durch den Chirurgen tatsächlich implantierten medizinischen Vorrichtung in der anatomischen Struktur,
- und Verwenden der 3D-Position und -Ausrichtung der durch den Chirurgen tatsächlich implantierten medizinischen Vorrichtung zum weiteren Trainieren des neuronalen Vorhersagenetzwerks (400), um eine Genauigkeit des neuronalen Vorhersagenetzwerks (400) zu verbessern, um anschließend eine bevorzugte Position und Ausrichtung zu identifizieren.

3. Verfahren nach Anspruch 1, ferner umfassend Verarbeiten der Abtastbilder der anatomischen Strukturen zwischen den identifizierten Orientierungspunkten und Bestimmen physischer Abmessungen der anatomischen Strukturen in der Region von Interesse, in der beabsichtigt ist, die medizinische Vorrichtung zu implantieren.

4. Verfahren nach Anspruch 3, ferner umfassend Bestimmen bevorzugter physischer Abmessungen, wobei die bevorzugten physischen Abmessungen mindestens eines von Folgenden enthalten: Durchmesser und/oder Länge der zu implantierenden medizinischen Vorrichtung in Abhängigkeit von analysierten Abmessungen der anatomischen Struktur.

5. Verfahren nach Anspruch 1, wobei die empfangenen medizinischen Abtastbilder von einem intraoperativen Abtaster gesammelt werden.

6. Verfahren nach Anspruch 1, wobei die empfangenen medizinischen Abtastbilder von einem präoperativen stationären Abtaster gesammelt werden.

7. Computerimplementiertes System, umfassend:
- mindestens ein nichtflüchtiges prozessorlesbares Speichermedium, das mindestens eines von prozessorausführbaren Anweisungen oder Daten speichert; und
- mindestens einen Prozessor, der kommunizierbar an mindestens ein nichtflüchtiges prozessorlesbares Speichermedium gekoppelt ist, wobei mindestens ein Prozessor dazu konfiguriert ist, die Schritte des Verfahrens nach einem der vorhergehenden Ansprüche durchzuführen.

## Revendications

1. Procédé permettant l'identification assistée par ordinateur d'une structure anatomique appropriée pour le placement d'un implant d'un dispositif médical dans la structure anatomique, dans lequel le dispositif médical est un implant chirurgical, comprenant les étapes suivantes :
- la réception d'un volume de balayage 3D comprenant un ensemble d'images de balayage médical d'une région d'une structure anatomique où le dispositif médical doit être implanté (301) ;
- le traitement automatique de l'ensemble d'images médicales de balayage pour obtenir des données de segmentation (302) ;
- la combinaison de l'ensemble d'images médicales de balayage avec les données de segmentation en images combinées (303) ;
- la détermination automatique d'une sous-section du volume de balayage 3D en tant que région d'intérêt (ROI) 3D (304) à partir des images combinées ;
- le traitement automatique de la ROI 3D pour identifier des points de repère au sein de la structure anatomique pour l'implantation du dispositif médical dans la structure anatomique à l'aide d'un réseau neuronal (400) convolutionnel, CNN, de prédiction pré-entraîné ;
- la détermination automatique, sur la base des points de repère, d'une position et d'une orientation 3D préférées du dispositif médical à implanter par rapport à la structure anatomique (309) ; et
- la détermination automatique d'une position et d'une orientation 3D préférées du dispositif médical à implanter par rapport au volume de balayage 3D de la structure anatomique (309).

2. Procédé selon la revendication 1, comprenant en outre :
- la réception, à partir d'une base de données d'entraînement, de données provenant d'une intervention chirurgicale effectivement réalisée impliquant l'implantation d'un dispositif médical par un chirurgien dans la structure anatomique au cours de l'intervention chirurgicale,
- l'identification et le stockage automatiques d'une position et d'une orientation 3D du dispositif médical effectivement implanté par le chirurgien dans la structure anatomique,
- et l'utilisation de la position et de l'orientation 3D du dispositif médical effectivement implanté par le chirurgien pour un entraînement supplémentaire du réseau neuronal (400) de prédiction afin d'améliorer une précision du réseau neuronal (400) de prédiction pour identifier ultérieurement une position et une orientation préférées.

3. Procédé selon la revendication 1, comprenant en outre le traitement des images de balayage des structures anatomiques entre les points de repère identifiés, et la détermination de dimensions physiques des structures anatomiques dans la région d'intérêt où le dispositif médical est destiné à être implanté.

4. Procédé selon la revendication 3, comprenant en outre la détermination de dimensions physiques préférées, les dimensions physiques préférées comprenant au moins un parmi : un diamètre et/ou une longueur, du dispositif médical à implanter en fonction de dimensions analysées de la structure anatomique.

5. Procédé selon la revendication 1, dans lequel les images de balayage médical reçues sont collectées à partir d'un scanner peropératoire.

6. Procédé selon la revendication 1, dans lequel les images de balayage médical reçues sont collectées à partir d'un scanner stationnaire pré-chirurgical.

7. Système mis en œuvre par ordinateur, comprenant :
- au moins un support de stockage lisible par processeur non transitoire qui stocke au moins l'une de données ou d'instructions exécutables par processeur ; et
- au moins un processeur couplé en communication à au moins un support de stockage lisible par processeur non transitoire, dans lequel au moins un processeur est configuré pour réaliser les étapes du procédé de l'une quelconque des revendications précédentes.
